# EUROPEAN PATENT SPECIFICATION

(11) **EP 1 937 214 B1**
(45) Date of publication and mention of the grant of the patent: **27.07.2016**
(21) Application number: 06789920.3
(22) Date of filing: 23.08.2006
(51) Int. Cl.: A61K 9/127, A61K 9/19

(54) **METHOD OF STORING NANOPARTICLE FORMULATIONS**
VERFAHREN ZUR AUFBEWAHRUNG VON NANOTEILCHENFÖRMIGEN FORMULIERUNGEN
PROCEDE POUR STOCKER DES PREPARATIONS DE NANOPARTICULES

(30) Priority: 23.08.2005 US 710156 P
(43) Date of publication of application: 02.07.2008
(73) Proprietor: Celsion Corporation, Columbia, MD 21046-1705 (US)
(72) Inventor: YU, Weiping, Clarksville, Maryland 21029 (US); MON, John, Silver Spring, Maryland 20905 (US)
(74) Representative: Rowe, Daniel
(86) International application number: PCT/US2006/032714
(87) International publication number: WO 2007/024826

(56) References cited:
- EP-A1- 1 541 228
- WO-A-99/65466
- WO-A-2004/019913
- WO-A1-97/42936
- WO-A2-96/20698
- MOHAMMAD RIAZ: "Stability and uses of liposomes", PAKISTAN JOURNAL OF PHARMACEUTICAL SCIENCES, vol. 8, no. 2, July 1995 (1995-07), pages 69-79,
- FRANSEN G J ET AL: "Critical parameters in freezing of liposomes", INTERNATIONAL JOURNAL OF PHARMACEUTICS, ELSEVIER BV, NL, vol. 33, no. 1-3, 1 November 1986 (1986-11-01), pages 27-35, XP025813016, ISSN: 0378-5173, DOI: 10.1016/0378-5173(86)90035-9 [retrieved on 1986-11-01]
- VAN BOMMEL E M G ET AL: "Stability of doxorubicin-liposomes on storage: as an aqueous dispersion, frozen or freeze-dried", INTERNATIONAL JOURNAL OF PHARMACEUTICS, ELSEVIER BV, NL, vol. 22, no. 2-3, 1 December 1984 (1984-12-01), pages 299-310, XP025524601, ISSN: 0378-5173, DOI: 10.1016/0378-5173(84)90030-9 [retrieved on 1984-12-01]

## Description

### BACKGROUND OF THE INVENTION

### Field of the Invention

The present invention generally relates to a method of storing nanoparticles. More particularly, the invention relates to a method of storing liposomes having enhanced stability and storage characteristics.

### Background Information

Several standard techniques have been used for liposome and nanoparticle storage, such as refrigeration, freeze/drying (or lyophilization), and dehydration. In the lyophilization process, preservation fillers (cryoprotectants), such as sugars and sugar derivatives, can be used to enhance storage. These preservation fillers help, but do not always adequately overcome the problems of stability or damage of the liposomes during freezing and thawing processes. There are also limitations as to economic and commercial efficiency of the current processes since incorporating such preservation fillers can lead to limited shelf life under refrigeration or freezing conditions. Further, detrimental changes to the biological, chemical, and/or physical properties of the encapsulated active agents can occur during the freezing, thawing and/or drying processes or the dehydration process associated with lyophilization. Current lyophilization processes (the freezing and drying cycles) can be also very slow and time-consuming. A significant concern is that if lyophilization is performed too rapidly, or at uncontrolled temperatures, it may result in changes to the biological, chemical, and/or physical characteristic of the liposome membrane resulting in instability on storage. Ultimately, this may also detrimentally affect the properties of the encapsulated active agent.

During conventional freezing processes, used alone or in combination with other processes, damage may be caused, for example, by mechanical stress due to high pressure as a result of the liposomal vesicle membranes being exposed to ice crystal formation or by the expansion and contraction of solutes within the liposomes during freezing, thawing, and/or dehydration. The expansion of the liposome membrane during a freezing cycle can cause weakness, and the formation of fistulas and cracks in the liposome membrane, which, in turn, results in loss of integrity and stability leading to leakage problems. In addition, individual liposomes are normally closely bundled with each other, such that and during conventional freezing, the expansion of one liposome can cause pressure that results in adverse consequences to the integrity of adjacent liposomes.

Van Bommel et al report on the stability of doxorubicin-liposomes on storage as an aqueous dispersion, frozen or freeze-dried (Van Bommel, Crommelin; International Journal of Pharmaceutics, 22 (1984) 299-310).

Generally, existing technologies have their own limitations with regards to the shelf life, storage, handling, and entrapment properties of liposomes as well as the ability to maintain the integrity of the (i) liposome or nanoparticle composition; and (ii) active agent. In addition, many of the technologies have additional limitations due to the length of time required to perform the processes, and the resultant size of process production batches, which add to the overall costs.

### SUMMARY OF THE INVENTION

The present invention is a method of storing a liquid suspension liposome formulation having: at least one phospholipid, at least one surfactant, at least one hydrophilic polymer and an active agent, wherein: the phospholipid is at least one phosphatidyl choline; the surfactant is at least one lysolipid; and the hydrophilic polymer is chosen from DSPE-mPEG-2000, DSPE-mPEG-5000 and mixtures thereof, which comprises freezing said formulation and storing the frozen formulation for a period of at least one month.

### BRIEF DESCRIPTION OF THE FIGURES

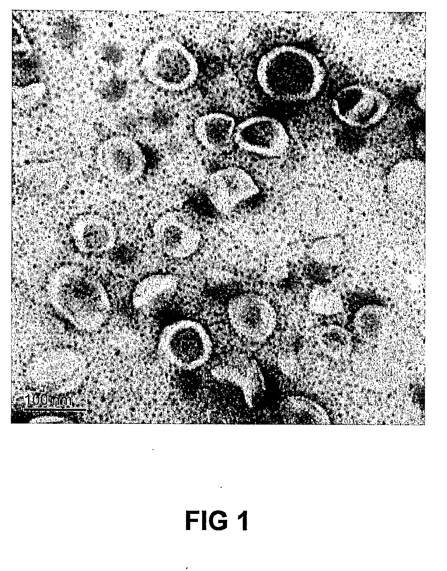

Figure 1 shows a liposome preparation after freezing and thawing according to the present invention.

### DESCRIPTION OF THE INVENTION

Embodiments of the invention are discussed below. In describing embodiments, specific terminology is employed for the sake of clarity. However, the invention is not intended to be limited to the specific terminology so selected. While specific exemplary embodiments are discussed, it should be understood that this is done for illustration purposes only.

As used herein, nanoparticles can be defined as generally spherical vesicles having an outer layer or membrane formed from an amphiphile, i.e., a component having a hydrophilic portion and a hydrophobic portion, that surrounds an inner core. In general, dimensions of nanoparticles are less than about 1000 nm. Of particular interest with respect to the present invention are nanoparticles that can be used in or as a drug delivery system. For example, nanoparticles useful in the present invention can be used to encapsulate compounds for delivery to a biological system which include, but are not limited to, (i) biologically active compounds, such as a drug; or (ii) non-biologically active compounds, such as an imaging agent. Liposomes are an exemplary type of nanoparticle that can be used with the present invention. Liposomes are vesicles which include a lipid bilayer surrounding an aqueous core, and which are also capable of encapsulating biologically active and non-biologically active compounds. The present invention enhances and protects the integrity of the nanoparticle or liposome membrane from damage caused by various stresses such as freezing, thawing and agitation. Thus, the invention enhances the stability, storage, and/or shelf life characteristics of nanoparticles, such as liposomes. As used herein, freezing can be at any temperature, generally less than 0°C, which provides a solid state, for example, about -20°C or below, about -78°C or below (i.e., dry ice temperatures) or about -196°C or below (i.e., liquid nitrogen temperatures).

Compositions of various liposomes and other nanoparticle formulations are well known in the art and are used commercially for medical, cosmetic and other purposes. Liposome formulations are combinations of various lipids in different molar ratios, as well as other constituents including, but not limited to, targeting moieties and biologically active substances. Liposomal production processes are generally known. Liposomes can be made in various sizes, e.g., from less than 20 nm to greater than 500 nm in diameter; and can be unilamellar or multilamellar vesicles. They can be used to encapsulate various substances used for medical and non-medical purposes. Liposomes can be used to entrap active agents such as pharmacologically active agents, flavor agents, diagnostic agents, nutritional agents, gene products, non-biologically active products such as imaging agents, and mixtures thereof, and to deliver these agents to specific sites. However, a major problem with liposome formulations has been the ability to store them for research or commercial purposes without degradation of the lipids and/or release of the active agents (e.g., shelf life). Thus, vesicle stability with respect to leakage and degradation of the liposome over time is a major problem.

An embodiment of the invention relates to storage of a nanoparticle formulation, for example a liposome formulation, but may be applicable for other types of nanoparticles composed of amphiphiles such as lipids. Formulations suitable for use in the present invention include nanoparticles having in the membrane components that include at least one amphiphile, at least one surfactant, and at least one hydrophilic polymer. The hydrophilic polymer may be unmodified; modified by the addition of functional groups to associated with the nanoparticle membrane; or chemically bound to an amphiphile, surfactant, or other component of the nanoparticle membrane. An example of a modification to associate the hydrophilic polymer with the nanoparticle membrane can include the addition of a polar or charge-forming functional group to produce a stronger association with the polar group of the membrane-forming amphiphile or surfactant. The hydrophilic polymer may, alternatively, be covalently bound to the membrane-forming amphiphile or surfactant so that it is present on the surface of the nanoparticle or liposome to protect and maintain its integrity, or it may be incorporated into the inner lipid layer of the liposome or nanoparticle formulation. The hydrophilic polymer can be bonded to the amphiphile or surfactant through a biodegradable or hydrolysable linkage such as, for example, an amide or ester linkage, or through a more stable linkage such as an amine or ether linkage.

Without being bound by theory, it is believed that the hydrophilic polymer affords protection of the integrity of the liposome or nanoparticle membrane during freezing, refrigeration, lyophilization, dehydration and/or rehydration processes. Protection of the integrity of the nanoparticle and liposomal membranes preserves the physical, chemical and biological properties of the nanoparticle and liposomal formulation, and also maintains the properties of any compound entrapped or encapsulated within the nanoparticle or liposome. Thus, the liposome or nanoparticle formulation preserves the entrapped encapsulates for medical, veterinary, and non-medical uses such as, but not limited to, the biotechnology, electronics, defense and agricultural arts and allows for storage and transport of liposomal formulations in the frozen state.

The ratio of amphiphile to surfactant is variable and can be any ratio that is suitable for the intended use of the nanoparticle or liposome. It is recognized that both the amphiphile and surfactant may have amphiphilic and surface active properties; however, when both components have similar properties, for the purposes of this specification, the amphiphile can be considered as the major membrane component, while the surfactant is the minor component. Thus the ratio of amphiphile to surfactant is at least about 51:49 and can be as large as 99:1. In exemplary embodiments, the amphiphile:surfactant ratio is at least about 70:30, and can be about 80:20 or 90:10. As is known in the art, the addition of surfactant can cause variations in the physical or chemical properties of the nanoparticle or liposome to achieve some desirable property such as a reduction in phase transition temperature or permeability of the membrane. Such variations in nanoparticle composition are within the scope of the invention.

The hydrophilic polymer may be present in the membrane of a nanoparticle or liposome in varying amounts ranging from about 0.1 to about 25 molar percent, based on the total amount of amphiphile and surfactant. In embodiments where the hydrophilic polymer is bound to a liposome-forming phospholipid that contains about 75-90% by weight hydrophilic polymer and 25-10% by weight lipid, this corresponds to about 0.11 to about 33 molar percent of hydrophilic polymer modified phospholipid. As will be appreciated by those skilled in the art, the conversion of molar percent hydrophilic polymer to molar percent of modified amphiphile will depend on the molecular weight of the unmodified amphiphile and the molecular weight of the hydrophilic polymer. Calculation of the ratios is well within the purview of persons skilled in the art. Embodiments of the invention can include about 3 to 20 molar percent hydrophilic polymer (for example, from about 3 to about 27 molar percent modified amphiphile) or 3 to 10 molar percent hydrophilic polymer (for example, from about 3 to about 13 molar percent modified amphiphile). Certain embodiments of the invention are prepared from about 4 to about 5 molar percent modified amphiphile, thus providing about from about 2.5 to about 4.5 molar percent hydrophilic polymer. The optimal amount of hydrophilic polymer can be determined easily through routine experimentation by persons of skill in the art.

The compositions and methods of the invention can be effective with nanoparticles and liposomes prepared from a wide variety of compositions including, but not limited to phospholipids, soybean lipids, phosphoethanolamines, cholesterol, lysolipids, surfactants, and mixtures thereof, in combination with at least one hydrophilic polymer such as polyethylene glycol, polyacryloylmorpholine, poly-2-ethyl-2-oxazoline, polyvinylpyrrolidone, methoxypolyethylene glycol (mPEG) derivatives and mixtures thereof. Examples of the phospholipids of the invention include, but are not limited to, phosphatidyl cholines, phosphatidyl glycerols, phosphatidyl inositols, phosphatidyl ethanolamines, and sphingomyelins. Representative surfactants useful in the invention can include, but are not limited to, dichain phospholipids, lysolipids, bile acids, myristoyl surfactants, palmitoyl surfactants, stearoyl surfactants, glyceryl monooleates, ceramides, PEG-ceramides, C18-ether linked lysophosphatidyl choline, polyethylene glycol-polyethylene copolymers, block copolymers, fatty acids and mixtures thereof. Examples of lysolipids useful in the invention include, but are not limited to monopalmitoyl phosphatidylcholine (MPPC), monolauryl phosphatidylcholine (MLPC), monomyristoyl phosphatidylcholine (MMPC), monostearoyl phosphatidylcholine (MSPC), and mixtures thereof. Representative hydrophilic polymers useful in the invention include, but are not limited to, polyethylene glycol, polyacryloylmorpholine, poly-2-ethyl-2-oxazoline, polyvinylpyrrolidone, and methoxypolyethylene glycol (mPEG), and mixtures thereof. Examples of active agents useful in the invention include, but are not limited to, pharmacologically active agents, flavor agents, diagnostic agents, nutritional agents, gene products, non-biologically active products such as imaging agents, and mixtures thereof. Exemplary types of active agents include anesthetics, anti-histamines, anti-neoplastics, anti-ulceratives, anti-seizure agents, muscle relaxants, immunosuppressive agents, anti-infective agents, non-steroidal anti-inflammatory agents, imaging agents, nutritional agents, and mixtures thereof, for example. Exemplary anti-neoplastic agents include, but are not limited to, anthracyclines, such as doxorubicin and epirubicin; taxanes, such as taxol and taxotere; and platins, such as cis-platin, carboplatin and oxaliplatin.

One embodiment of the invention utilizes a liposome formulation comprising the phospholipid DPPC, the lysolipid MSPC, and a phosphoplipid such as 1,2-distearoyl-sn-glycero-3-phosphoethanolamine (DSPE) functionalized to include a hydrophilic polymer, for example DSPE-mPEG-2000 and/or DSPE-mPEG-5000, in which the hydrophilic polymer is attached to the lipid through an amide linkage. Liposome compositions comprising molar ratios of phospholipid:lysolipid:hydrophilic polymer functionalized-phosphoplipids as low as 90:10:1 have been shown to be effective in the method of freezing and storing. Consequently, this invention has a broad-based application which can be used to increase efficiency and lower process costs for commercial liposomal products. This invention can be applied to a liposomal
formulation according to claim 1 to protect the integrity of the liposome during freezing.

In one embodiment, the invention relates to a method of storing a nanoparticle formulation, for example a liposomal formulation, with enhanced properties such as stability, storage, entrapment, or release characteristics. It has been found that the incorporation of hydrophilic polymers into the membrane of nanoparticles, such as liposomes enhances the stability, storage, entrapment, and release characteristics of such vesicles. The inclusion of hydrophilic polymers in nanoparticle and liposomal formulations limits the detrimental changes to the biological, chemical, and physical properties of the nanoparticle or liposomal membrane caused by freezing, refrigeration, thawing, lyophilization, dehydration or other methods used to aid in the storage, stability, and handling of said formulations. Thus, the presence of a hydrophilic polymer in association with the nanoparticle or liposomal formulation protects, maintains and/or enhances the integrity of the original membrane.

In the past, it has not been possible to effectively use freezing alone for the purpose of storing liposomes. The majority of liposome products have warning labels which state, "Do Not Freeze." Examples of this can be seen on labels of commercially-available products such as Doxil, AmBisome, DepoCyt, and several cosmetic liposomes. A formulation useful with the present invention can provide a composition wherein liposomes and nanoparticles can be frozen without damaging the membrane integrity or the encapsulated contents. Thus, employing the formulations of the invention would allow for single vial and combinations of multi-vial products, to be frozen and stored for distribution purposes. That is, a particular advantage of the present invention is that a liposome formulation can be frozen and transported in a single container, which may be in unit dosage form, rather than being shipped in separate containers that require re-formulation immediately prior to use.

The invention also includes a method for formulating and storing a nanoparticle or liposomal preparation, the method includes combining amphiphiles or lipids suitable for the formation of a nanoparticle or liposomal composition with a hydrophilic polymer, freezing and storing. Such nanoparticles and liposomes can be formed using methods generally known in the art; e.g., by mixing the amphiphilic components with an aqueous solution under suitable conditions. Suitable methods are well known to those of skill in the art. See; e.g., Needham, U.S. Patent No. 6,200,598 and 6,726,925; Ogawa, U.S. Patent No. 5,094,854. The nanoparticle and liposomal compositions may be stored for at least one month.

Furthermore, the method described allows for the nanoparticle and liposomal formulations to be dispensed into unit doses in multiple or single vials prior, or subsequent, to freezing and storing. The nanoparticle and liposomal formulations of the invention can encapsulate at least one of: cytotoxic and non-cytotoxic drugs, gene products, or biological agents, as well as non-biological agents, e.g., imaging agents, that can be used immediately or frozen packaged as kits.

Another concern during the use of nanoparticle and liposomal compositions in humans and/or animals, is that the compositions may be recognized and eliminated by natural body defenses, such as the reticuloendothelial system (RES), that limit their effective circulation time and therapeutic utility. Several methods have been examined, such as optimizing the size of the nanoparticle and liposomal compositions or by making "stealth" compositions by adding certain constituents to the composition membranes to protect or "hide" them from natural biological elimination processes. During storage, nanoparticles and liposomes have a tendency to increase in size which results in a loss of membrane integrity and stability. Developing a method to maintain the desired nanoparticular and liposomal size and uniformity, may help increase and preserve the effective circulation time in a subject, as an alternative to adding separate constituents specifically to produce such "stealth" characteristics. For this reason, protecting the size of the nanoparticle or liposome during freezing, refrigeration, dehydration or lyophilization, can be very important to their integrity and performance. Thus, a formulation which enhances the stability of the nanoparticle or liposomal can keep the particle size constant during storage and maintain its therapeutic effectiveness.

In an exemplary embodiment, an amphiphile, surfactant and an effective amount of hydrophilic polymer (or amphiphile modified to contain a hydrophilic polymer) are combined to form a nanoparticle using known methods. For example, the amphiphile, surfactant and hydrophilic polymer (or amphiphile modified to contain a hydrophilic polymer can be combined in an organic solvent, and the solvent removed, followed by the addition of an aqueous solution. An active agent can then be loaded into the interior space of the nanoparticle using known techniques such as, for example, the use of a pH gradient. The nanoparticles can be sized using known techniques such as, for example, extrusion through a suitable polycarbonate filter. Sizing can be conducted prior to or after the loading of the nanoparticle with an active agent. The final nanoparticle size can be less than about 1000 nm or from about 10 to about 500 nm, from about 25 to about 500 nm, from about 50 to about 200 nm or from about 80 to about 125 nm. The effective amount of hydrophilic polymer can be determined by preparing the nanoparticle, freezing at the desired temperature, storing for an appropriate amount of time, thawing the formulation and measuring a characteristic of the nanoparticle indicative of stability. The measured characteristic can be, for example, particle size or leakage of active agent. The amount of hydrophilic polymer in the initial preparation can then be varied to achieve the desired degree of stability.

After preparation of the nanoparticle, the formulation can be frozen and stored in a solid state at, for example, at about -20°C or below, about -78°C or below or about -196°C or below. In exemplary embodiments, the nanoparticles can be stored in the frozen state for about three or more days without a significant change in physical properties upon thawing. In other embodiments, the nanoparticle formulation can be stored for one month or longer, for about three months or longer or for about 24 months, or for an even more extended time. The formulation can be stored as a bulk solution or, prior to freezing, can be divided into unit doses for distribution. In addition to storage, the frozen nanoparticle formulation can be transported to the site where it will be used. For example, the formulation can be prepared at a production facility, separated into unit doses, frozen and shipped to a location where it will be used. This offers advantages over existing formulations and methods of distribution. Using traditional methods, nanoparticle formulations are often separated into component parts for shipping in order to prevent loss of stability. For example, liposomal formulations are often shipped in multiple vials, with the liposomes in one container and a solution of the active agent in another container. Prior to use, the two separate containers must be combined in such a way to ensure loading of the active agent prior to administration. Errors in preparing this final solution can result in a lack of effectiveness. Using the present invention, this combining step is eliminated. Thus, at the site of use, the formulation is thawed and can be used or administered directly. This facilitates use of the composition and ensures that the composition is prepared correctly for effective administration.

Thus, the present invention provides a method for storing, transporting and administering a nanoparticulate or liposomal formulation. Formulations stored and/or transported according to the present invention can be administered in any way that is useful for the particular formulation. Methods of administration include oral, rectal, peritoneal, intravenous and buccal administration, although any desirable route of administration may be utilized depending on the active agent and its site of activity. The formulation may be administered alone; i.e., as manufactured, or combined with other therapeutic agents or vehicles prior to use. Vehicles can include other solid or liquid excipients such as aqueous solutions, suspensions, emulsions, solid vehicles, dispersing agents, etc. Final application is consistent with the use of the nanoparticle formulation had it been utilized directly after preparation, i.e., before storage and transport.

These and other features and advantages of the invention will be further understood from the following representative example which is not intended to limit the scope of the present invention.

### REPRESENTATIVE EXAMPLE 1.

Dipalmitoyl-phosphatidylcholine (DPPC), 1-stearoyl-2-lyso-phosphatidylcholine (MSPC) and N-(carbonyl-methoxypolyethyleneglycol-2000)-1,2-distearoyl-sn-glycero-3-phospho-ethanolamine (DSPE-mPEG-2000) in a molar ratio of 90:10:4 were dissolved in dichloromethane. The organic solvent was then removed by rotary evaporation. The mixture was then hydrated using 300 mM citric buffer at pH 4 to obtain a final lipid concentration of about 100 mg/ml. The suspension formed was then extruded through two polycarbonate membrane filters. (Hope, M.J. et al Production of large unilamellar vesicles by a rapid extrusion procedure. characterization of size, trapped volume and ability to maintain a membrane potential. Biochim. Biophys. Acta. 812: 55-65, 1985) The final liposome formulations in this example had an initial mean particle size of between 98 and 122 nm. (In general, the particle size of the liposomes of the invention will be between 50-500 nm depending on the extrusion method.) The drug, doxorubicin, was loaded into the liposome by mixing the liposomes with sodium carbonate solution and the drug. Briefly, 1. 6 ml of doxorubicin solution (5.8 mg/ml) and 1.2 ml of 0.5 mM sodium carbonate were added to 1.9 ml of the liposome mixture and equilibrated at 35°C for 60 minutes. The drug-loaded liposomes, as well as empty liposomes, were then stored at -20°C. The change in particle size was monitored. Liposome compositions, without the addition of mPEG, were made by the same process. Table 1 shows that with the addition of mPEG, the liposome particle size remains the same after freezing and thawing, and that the liposome is stable for up to 24 months as measured by standard assays of liposome integrity and stability. By contrast, liposomes which lack the hydrophilic polymer mPEG, are not stable after freezing and thawing. Evidence of such instability is that the particle size increases after freezing. Figure 1 shows a liposome preparation post freeze/thaw. There is no significant difference in the visual appearance and particle size pre- and post-freeze/thaw.

**Table 1. Liposome particle size change after freezing and thawing**

| Time | Particle Size (nm) | | | |
|---|---|---|---|---|
| | Liposome with MPEG | | Liposome without MPEG | |
| | Empty liposome | Doxorubicin liposome | Empty liposome | Doxorubicin liposome |
| Initial | 98.5 | 107 | 122 | 121 |
| 3 days | Not Tested | Not Tested | 131 | 137 |
| 3 Months | 100 | 105 | Not Tested | Not Tested |
| 24 months | 99.6 | Not Tested | Not Tested | Not Tested |

### REPRESENTATIVE EXAMPLE 2

Another doxorubicin-containing liposome preparation was prepared as in Representative Example 1.
Samples were frozen at -20°C for 1 week, 1 month and 3 months. At each time point, doxorubicin content, % recovery at 1.2 µm, % encapsulation, lipids concentrations, pH and vesicle size were determined. At the initial time point (t = 0), three samples were tested with or without freezing to determine the impact of freezing on the liposome formulation. In addition, one sample was exposed to three freeze-thaw cycles to determine if multiple freeze-thaw cycles would be detrimental.

As can be seen in the table below, at t=0 the liposome formulations had similar characteristics at ambient or frozen conditions. Even three freeze-thaw cycles did not impact membrane integrity. The formulations were stable over a 3-month storage period at -20°C. No significant changes in vesicle size, doxorubicin content, encapsulation or lipids content were observed over this time frame. Although the measured external pH of the samples varied slightly at the different time points, pH measurements of liposomes prepared in carbonate buffer have been observed to change with time as CO₂ gas escapes from the vial. However, as illustrated in Table 2, the changes in pH do not affect membrane stability as the 3-month pH data is similar to that at week one.

**Table 2. Liposome stability after repeated freezing and thawing**

| | **t=0 Not Frozen** | **t=0 Frozen** | **t=0 Frozen/Thawed 3 times** | **1 Week** | **1 Month** | **3 Month** |
|---|---|---|---|---|---|---|
| DOX (mg/ml) | 1.85 | 1.76 | 1.77 | 1.83 | 1.76 | 1.73 |
| % Recovery 1.2um | 99.2 | 99.8 | 99.9 | 100.2 | 99.3 | 100.5 |
| % Encapsulation | 99.9 | 99.5 | 99.4 | 99.5 | 99.5 | 98.2 |
| Total Lipids (mg/ml) | 35.1 | 35.2 | 35.0 | 34.9 | 34.9 | 35.7 |
| pH | 8.15 | 7.85 | 7.85 | 8.50 | 8.84 | 8.52 |
| Size | 97.0 | 98.0 | 98.3 | 98.9 | 96.7 | 97.8 |

The embodiments illustrated and discussed in this specification are intended only to teach those skilled in the art the best way known to the inventors to make and use the invention. Nothing in this specification should be considered as limiting the scope of the present invention. All examples presented are representative and non-limiting. The above-described embodiments of the invention may be modified or varied, without departing from the invention, as appreciated by those skilled in the art in light of the above teachings. It is therefore to be understood that, within the scope of the claims and their equivalents, the invention may be practiced otherwise than as specifically described.

## Claims

1. A method of storing a liquid suspension liposome formulation having:
at least one phospholipid, at least one surfactant, at least one hydrophilic polymer and an active agent, wherein:
the phospholipid is at least one phosphatidyl choline;
the surfactant is at least one lysolipid; and
the hydrophilic polymer is chosen from DSPE-mPEG-2000, DSPE-mPEG-5000 and mixtures thereof,
which comprises freezing said formulation and storing the frozen formulation for a period of at least one month.

2. The method of claim 1, wherein the phospholipid is at least one phosphatidyl choline; the surfactant is at least one chosen from monopalmitoyl phosphatidylcholine (MPPC), monolauryl phosphatidylcholine (MLPC), monomyristoyl phosphatidylcholine (MMPC), monostearoyl phosphatidylcholine (MSPC), and mixtures thereof; and the hydrophilic polymer is chosen from DSPE-mPEG-2000 or DSPE-mPEG-5000 and mixtures thereof.

3. The method of claim 2, wherein the phospholipid is dipalmitoyl phosphatidylcholine (DPPC); the surfactant is monopalmitoyl phosphatidylcholine (MPPC); and the hydrophilic polymer is chosen from DSPE-mPEG-2000, DSPE-mPEG-5000 and mixtures thereof.

4. The method of claim 2, wherein the phospholipid is dipalmitoyl phosphatidylcholine (DPPC); the surfactant is monostearoyl phosphatidylcholine (MSPC); and the hydrophilic polymer is chosen from DSPE-mPEG-2000, DSPE-mPEG-5000 and mixtures thereof.

5. The method of claim 3, wherein the molar ratio of DPPC:MPPC:DSPE-mPEG is about 90:10:4.

6. The method of claim 4, wherein the molar ratio of DPPC:MSPC:DSPE-mPEG is about 90:10:4.

7. The method of any one of claims 1 to 6, wherein the active agent is chosen from pharmacologically active agents, flavor agents, diagnostic agents, nutritional agents, gene products, and mixtures thereof.

8. The method of claim 7, wherein the active agent is chosen from anesthetics, antihistamines, anti-neoplasties, anti-ulceratives, anti-seizure agents, muscle relaxants, immunosuppressive agents, anti-infective agents, non-steroidal anti-inflammatory agents, imaging agents, nutritional agents, and mixtures thereof.

9. The method of claim 8, wherein the active agent is at least one anti-neoplastic agent.

10. The method of claim 9, wherein the active agent is doxorubicin.

11. The method of any one of claims 1 to 10 which further comprises transporting the frozen liposome formulation and thawing said formulation at another location.

12. The method of claim 1, wherein the at least one phosphatidyl choline is dipalmitoyl phosphatidylcholine (DPPC), the at least one lysolipid is monostearoyl phosphatidylcholine (MSPC), the hydrophilic polymer is DSPE-mPEG-2000, and the active agent is doxorubicin, wherein the molar ratio of DPPC:MSPC:DSPE-mPEG-2000 is 90:10:4.

## Patentansprüche

1. Verfahren zum Lagern einer flüssigen Suspension einer Liposomenformulierung enthaltend:
wenigstens ein Phospholipid, wenigstens ein Tensid, wenigstens ein hydrophiles Polymer und einen Wirkstoff, wobei
das Phospholipid wenigstens ein Phosphatidylcholin ist;
das Tensid wenigstens ein Lysolipid ist; und
das hydrophile Polymer ausgewählt ist aus DSPE-mPEG-2000, DSPE-mPEG-5000 und Mischungen davon,
welches ein Einfrieren der Formulierung und Lagern der gefrorenen Formulierung für einen Zeitraum von wenigstens einem Monat umfasst.

2. Verfahren nach Anspruch 1, wobei das Phospholipid wenigstens ein Phosphatidylcholin ist; das Tensid wenigstens eines ist, welches ausgewählt ist aus Monopalmitoylphosphatidylcholin (MPPC), Monolaurylphosphatidylcholin (MLPC) Monomyristoylphosphatidylcholin (MMPC), Monostearoylphosphatidylcholin (MSPC) und Mischungen davon; und das hydrophile Polymer ausgewählt ist aus DSPE-mPEG-2000 oder DSPE-mPEG-5000 und Mischungen davon.

3. Verfahren nach Anspruch 2, wobei das Phospholipid Dipalmitoylphosphatidylcholin (DPPC) ist; das Tensid Monopalmitoylphosphatidylcholin (MPPC) ist; und das hydrophile Polymer ausgewählt ist aus DSPE-mPEG-2000, DSPE-mPEG-5000 und Mischungen davon.

4. Verfahren nach Anspruch 2, wobei das Phospholipid Dipalmitoylphosphatidylcholin (DPPC) ist; das Tensid Monostearoylphosphatidylcholin (MSPC) ist; und das hydrophile Polymer ausgewählt ist aus DSPE-mPEG-2000, DSPE-mPEG-5000 und Mischungen davon.

5. Verfahren nach Anspruch 3, wobei das Molverhältnis von DPPC:MPPC:DSPE-mPEG ungefähr 90:10:4 ist.

6. Verfahren nach Anspruch 4, wobei das Molverhältnis von DPPC:MSPC:DSPE-mPEG ungefähr 90:10:4 ist.

7. Verfahren nach einem der Ansprüche 1 bis 6, wobei der Wirkstoff ausgewählt ist aus Pharmazeutika, Geschmacksstoffen, Diagnostika, Nahrungsmittel, Genprodukten und Mischungen davon.

8. Verfahren nach Anspruch 7, wobei der Wirkstoff ausgewählt ist aus Anästhetika, Antihistaminika, Antineoplastika, antiulzerativen Mitteln, Antikrampfmitteln, Muskelrelaxantien, Immunsuppresiva, Antiinfektiva, nicht-steroidalen entzündungshemmenden Mitteln, Kontrastmitteln, Nutrazeutika und Mischungen davon.

9. Verfahren nach Anspruch 8, wobei der Wirkstoff wenigstens ein Antineoplastikum ist.

10. Verfahren nach Anspruch 9, wobei der Wirkstoff Doxorubicin ist.

11. Verfahren nach einem der Ansprüche 1 bis 10, welches ferner ein Transportieren der gefrorenen Liposomenformulierung und ein Auftauen der Formulierung an einem anderen Ort umfasst.

12. Verfahren nach Anspruch 1, wobei das wenigstens eine Phosphatidylcholin Dipalmitoylphosphatidylcholin (DPPC) ist, das wenigstens eine Lysolipid Monostearoylphosphatidylcholin (MSPC) ist, das hydrophile Polymer DSPE-mPEG-2000 ist, und der Wirkstoff Doxorubicin ist, wobei das Molverhältnis von DPPC:MSPC:DSPE-mPEG-2000 90:10:4 ist.

## Revendications

1. Procédé de stockage d'une formulation liposomique en suspension liquide ayant :
au moins un phospholipide, au moins un surfactant, au moins un polymère hydrophile et un agent actif, dans lesquels :
le phospholipide est au moins une phosphatidylcholine ;
l'agent de surface est au moins un lysolipide ; et
le polymère hydrophile est choisi parmi DSPE-mPEG-2000, DSPE-mPEG-5000 et des mélanges de ceux-ci,
qui comprend la congélation de ladite formulation et le stockage de la formulation congelée pendant une durée d'au moins un mois.

2. Procédé selon la revendication 1, dans lequel le phospholipide est au moins une phosphatidylcholine ; le surfactant est au moins un choisi parmi la monopalmitoylphosphatidylcholine (MPPC), la monolaurylphosphatidylcholine (MLPC), la monomyristoylphosphatidylcholine (MMPC), la monostéaroylphosphatidylcholine (MSPC) et des mélanges de celles-ci ; et le polymère hydrophile est choisi parmi DSPE-mPEG-2000 ou DSPE-mPEG-5000 et des mélanges de ceux-ci.

3. Procédé selon la revendication 2, dans lequel le phospholipide est de la dipalmitoylphosphatidylcholine (DPPC) ; le surfactant est de la monopalmitoylphosphatidylcholine (MPPC) ; et le polymère hydrophile est choisi parmi DSPE-mPEG-2000, DSPE-mPEG-5000 et des mélanges de ceux-ci.

4. Procédé selon la revendication 2, dans lequel le phospholipide est de la dipalmitoylphosphatidylcholine (DPPC) ; le surfactant est de la monostéaroylphosphatidylcholine (MSPC) ; et le polymère hydrophile est choisi parmi DSPE-mPEG-2000, DSPE-mPEG-5000 et des mélanges de ceux-ci

5. Procédé selon la revendication 3, dans lequel le rapport molaire de DPPC:MPPC:DSPE-mPEG est d'environ 90:10:4.

6. Procédé selon la revendication 4, dans lequel le rapport molaire de DPPC:MSPC:DSPE-mPEG est d'environ 90:10:4.

7. Procédé selon l'une quelconque des revendications 1 à 6, dans lequel l'agent actif est choisi parmi des agents actifs d'un point de vue pharmacologique, des agents de saveur, des agents de diagnostic, des agents alimentaires, des produits génétiques et des mélanges de ceux-ci.

8. Procédé selon la revendication 7, dans lequel l'agent actif est choisi parmi des anesthésiques, des antihistaminiques, des anticancéreux, des agents antiulcéreux, des anticonvulsivants, des tranquillisants musculaires, des immunosuppresseurs, des agents anti-infectieux, des agents anti-inflammatoires non stéroïdiens, des agents d'imagerie, des agents alimentaires et des mélanges de ceux-ci.

9. Procédé selon la revendication 8, dans lequel l'agent actif est au moins un agent anticancéreux.

10. Procédé selon la revendication 9, dans lequel l'agent actif est de la doxorubicine.

11. Procédé selon l'une quelconque des revendications 1 à 10 qui comprend en outre le transport de la formulation liposomique congelée et le dégel de ladite formulation à un autre emplacement.

12. Procédé selon la revendication 1, dans lequel l'au moins une phosphatidylcholine est de la dipalmitoylphosphatidylcholine (DPPC), l'au moins un lysolipide est de la monostéaroylphosphatidylcholine (MSPC), le polymère hydrophile est du DSPE-mPEG-2000, et l'agent actif est de la doxorubicine, dans lequel le rapport molaire de DPPC:MSPC:DSPE-mPEG-2000 est 90:10:4.
